# EUROPÄISCHE PATENTANMELDUNG

(11) **EP 4 559 394 A1**
(43) Veröffentlichungstag der Anmeldung: **28.05.2025**
(21) Anmeldenummer: 23211909.9
(22) Anmeldetag: 24.11.2023
(51) Int. Cl.: A61B 5/22

(54) **VORRICHTUNG ZUR BESTIMMUNG EINER SCHLAGEIGENSCHAFT MIT ZWEI BESCHLEUNIGUNGSSENSOREN**

(71) Anmelder: Research Industrial Systems Engineering (RISE) Forschungs-, Entwicklungs- und Grossprojektberatung GmbH, 2320 Schwechat (AT)
(72) Erfinder: HÖLBLING, Dominik, 2482 Münchendorf (AT); GRECHENIG, Thomas, 1040 WIen (AT)
(74) Vertreter: Schwarz & Partner Patentanwälte GmbH

(57) **Zusammenfassung**

Die Erfindung betrifft eine energiesparoptimierte Vorrichtung (1) zur Bestimmung einer Schlageigenschaft, insbesondere einer Schlagbeschleunigung, Schlaggeschwindigkeit, Schlagkraft oder Schlagtechnik, umfassend eine Recheneinheit (4), einen ersten Beschleunigungssensor (2) und zumindest einen zweiten Sensor, der bevorzugt entweder ein zweiter Beschleunigungssensor (3) oder ein Drucksensor (300) in einem fluidgefülltem Körper (200) ist, wobei die Recheneinheit (4) sowohl mit dem ersten Beschleunigungssensor (2) als auch mit dem zweiten Sensor verbunden ist, wobei die Recheneinheit (4) und/oder der zweite Sensor als schaltbare Komponente ausgeführt und von einem ersten Betriebsmodus in einen zweiten Betriebsmodus versetzbar ist, wobei die schaltbare Komponente im ersten Betriebsmodus einen geringeren Strombedarf aufweist als im zweiten Betriebsmodus, und wobei der erste Beschleunigungssensor (3) oder der zweite Sensor dazu ausgebildet ist, die schaltbare Komponente vom ersten Betriebsmodus in den zweiten Betriebsmodus zu versetzen, wenn der erste Beschleunigungssensor (2) oder der zweite Sensor Messwerte mit einer vorbestimmten Eigenschaft liefert.

## Beschreibung

Die Erfindung betrifft eine Vorrichtung zur Bestimmung einer Schlageigenschaft, insbesondere einer Schlagbeschleunigung, Schlaggeschwindigkeit, (zumindest einer Kalkulation, basierend auf einer auf einer Schätzungen der "effektiven" Masse des Athleten beruhenden) Schlagkraft oder Schlagtechnik, umfassend eine Recheneinheit, einen ersten Beschleunigungssensor und einen zweiten Beschleunigungssensor.

Bei Kampfsportarten treten üblicherweise zwei oder mehr Athleten in einem Ring gegeneinander an und versuchen, mittels Schlägen, Tritten oder sonstigen Körperkontakten Treffer am jeweils anderen Athleten zu landen. Beispiele für derartige Kampfsportarten, die Gegenstand dieser Beschreibung sind, sind Boxen, Karate, Kickboxen, Taekwondo, Kung Fu etc.

Zu Wettkampfzwecken aber auch für Trainings und sonstige Tests ist es wünschenswert, einen Schlag oder Tritt oder deren Wirkung auf ein Körperteil zu klassifizieren, z.B. indem man dem Schlag oder Tritt eine Schlagfrequenz, Beschleunigung, Kraft, einen von der Beschleunigung oder der Kraft abgeleiteten Wert, oder eine daraus kombinierte bzw. aus einer Analyse abgeleitete Variable, wie die Schlagtechnik zuordnet. Zur Messung der Beschleunigung sind verschiedene Varianten bekannt, z.B. mittels einer Videoauswertung der Bewegung der Athleten oder mittels eines in einem Schlaghandschuh verbauten Trägheitssensors (Inertial Measurement Unit, IMU). Stellvertretend werden hierfür die Schriften US 2017/134712, die US 2018/001141, die US 2012/144414 und die WO 2019/106672 genannt.

In der Studie "Walilko, T. J., Viano, D. C., & Bir, C. A. (2005). Biomechanics of the head for Olympic boxer punches to the face. British journal of sports medicine, 39(10), 710-719" wurden Kräfte von Schlägen auf den Kopf eines Dummys gemessen (-78 g in kombinierter Richtung, z.B. 2 Achsen). Es wäre vorteilhaft, in der Praxis auftretende Messwerte zu erhalten, um Korrelationen mit Verletzungen oder die Leistungsfähigkeit der Athleten festzustellen.

Aus dem Stand der Technik ist weiters die US2011159939A1 bekannt, welche Beschleunigungssensoren mit einem Messbereich bis 8 g offenbart. Die Messwerte des Beschleunigungssensors werden ausgewertet, um daraus eine Schlagkraft zu berechnen.

Weiters ist die US 2010/0144414 A1 bekannt, die lehrt, dass einerseits Beschleunigungssensoren mit niedrigen Messbereichen und hohen Messraten und andererseits Beschleunigungssensoren mit hohen Messbereichen und niedrigen Messraten gleichzeitig eingesetzt werden können, um alle bei einem Schlag auftretenden Beschleunigungsmesswerte hinreichend gut zu bestimmen. Einerseits besteht jedoch das Problem, dass zum Messen des Schlagaufpralls gerade im hohen Messbereich eine hohe Messrate gefordert wird, was mit der vorgeschlagenen Lösung nicht erreicht wird. Andererseits besteht auch der große Nachteil, dass es zu einem sehr hohen Stromverbrauch kommt, da zwei Beschleunigungssensoren gleichzeitig ausgelesen und ausgewertet werden. Dadurch weist der Schlaghandschuh eine deutlich reduzierte Betriebsdauer auf. Die verbundene Batterie muss somit bereits nach kurzer Zeit wieder nachgeladen oder durch eine vollgeladene Batterie getauscht werden. Ziel ist es jedoch, dass der Schlaghandschuh für zumindest einen gesamten Wettkampftag, d.h. mehrere Stunden, benutzt werden kann, ohne dass die Batterie gewechselt oder nachgeladen werden muss.

Eine naheliegende Lösung für dieses Problem ist, eine Batterie mit höherer Kapazität einzusetzen, wobei dies jedoch wiederum das Gewicht des Schlaghandschuhes und das Volumen der Messeinheit erhöht, was nicht vom Athleten erwünscht ist.

Die Erfindung hat sich daher die Aufgabe gestellt, eine Vorrichtung zur Bestimmung einer Schlageigenschaft bereitzustellen, die Beschleunigungsmesswerte über einen großen Messbereich aufzeichnen und gleichzeitig über einen langen Betriebszeitraum eingesetzt werden kann und trotzdem ein geringes Volumen und Gewicht aufweist.

Diese Aufgabe wird erfindungsgemäß durch eine Vorrichtung zur Bestimmung einer Schlageigenschaft, insbesondere einer Schlagbeschleunigung, Schlaggeschwindigkeit, Schlagkraft oder Schlagtechnik, umfassend eine Recheneinheit, einen ersten Beschleunigungssensor und einen zweiten Sensor, der z.B. ein zweiter Beschleunigungssensor oder ein Drucksensor in einem fluidgefülltem Körper ist, wobei die Recheneinheit sowohl mit dem ersten Beschleunigungssensor als auch mit dem zweiten Sensor verbunden ist, wobei der zweite Sensor und/oder die Recheneinheit als schaltbare Komponente ausgeführt und von einem ersten Betriebsmodus in einen zweiten Betriebsmodus versetzbar ist, wobei die schaltbare Komponente im ersten Betriebsmodus einen geringeren Strombedarf aufweist als im zweiten Betriebsmodus, und wobei der erste Beschleunigungssensor oder der zweite Sensor dazu ausgebildet ist, die schaltbare Komponente vom ersten Betriebsmodus in den zweiten Betriebsmodus zu versetzen, wenn der erste Beschleunigungssensor oder der zweite Sensor Messwerte mit einer vorbestimmten Eigenschaft liefert, die insbesondere dadurch gegeben sein kann, dass die Beschleunigungsmesswerte des ersten Beschleunigungssensors einen Schwellwert überschreiten oder dass die Messwerte einer vorbestimmten Kurvencharakteristik entsprechen. Der Begriff "überschreiten" versteht sich hierin z.B. bezüglich der Absolutwerte der gemessenen Beschleunigung pro Richtung x, y, z separat oder über alle Richtungen zusammen als sqrt(x²+y²+z²).

Erfindungsgemäß kann eine Vorrichtung zur Schlagkraftbestimmung mit zumindest drei Komponenten (Beschleunigungssensor, weiterer Sensor, Recheneinheit) betrieben werden, wobei diese Vorrichtung einen reduzierten Strombedarf aufweist, indem zumindest eine der Komponenten als schaltbare Komponente ausgeführt wird und nur selektiv hinzugeschalten wird. Die Bedingung zum Versetzen der schaltbaren Komponente ist erfindungsgemäß dadurch gegeben, dass Messwerte eine vorbestimmte Eigenschaft aufweisen, z.B. einen Schwellwert überschreiten.

Im Regelfall wird die Vorrichtung derart ausgeführt, dass die Recheneinheit die schaltbare Komponente ist und vom zweiten Sensor, der z.B. als Hochbeschleunigungssensor ausgeführt ist, durch ein Interrupt-Signal - das bei einer Schwellwertüberschreitung ausgegeben wird - in den zweiten Betriebsmodus versetzt wird. Die Recheneinheit kann zu diesem Zeitpunkt einen internen Speicher eines Niedrigbeschleunigungssensors auslesen. Somit kann die Recheneinheit vergangene Daten vom Niedrigbeschleunigungssensor und zukünftige Daten vom Hochbeschleunigungssensor empfangen. Es sind jedoch auch andere Varianten möglich, z.B. indem ein Beschleunigungssensor den anderen in den zweiten Betriebsmodus versetzt. Diese Möglichkeiten werden unten näher erläutert.

Die erfindungsgemäße Vorrichtung ermöglicht, dass alle Messbereiche mit dem jeweils dafür vorgesehenen Beschleunigungssensor/Sensor erfasst werden können, wobei gleichzeitig ein geringer Strombedarf vorliegt. Erfindungsgemäß wird die schaltbare Komponente somit nur hinzugeschalten, wenn mit hoher Wahrscheinlichkeit ein Schlag vorliegt, und kann danach wieder weggeschalten werden.

Überraschenderweise war es möglich, die schaltbare Komponente auch "on the fly" während eines Schlages hinzuzuschalten. Aufgrund der kurzen Aufpralldauern schien dies eingangs zwar kontraintuitiv, jedoch hat sich in Versuchen herausgestellt, dass die Komponenten hinreichen schnelle Reaktionszeiten haben, um "on the fly" hinzugeschalten werden zu können.

Mit der erfindungsgemäßen Lösung kann somit die Batterielaufzeit der Vorrichtung erhöht werden, was essenziell ist, wenn die Vorrichtung über einen längeren Zeitraum - wie z.B. einen gesamten Wettkampftag - betrieben werden soll. Würde die erfindungsgemäße Lösung nicht eingesetzt werden, müsste eine größere Batterie eingesetzt werden, um eine entsprechende Vorrichtung über einen gesamten Wettkampftag zu betreiben. In einer alternativen Sichtweise kann die Erfindung somit auch als Gewichtsreduktion der Vorrichtung angesehen werden, da eine entsprechend kleinere Batterie eingesetzt werden kann.

Der eingangs genannte Schwellwert ist ein Beschleunigungswert, ab dem die schaltbare Komponente bzw. Komponenten hinzugeschalten werden soll bzw. sollen. Alternativ zu einem Schwellwert kann auch eine vorbestimmte Kurvencharakteristik erkannt werden, z.B. ein sprunghafter Anstieg der Beschleunigungsdaten, da dies auf einen Schlag hindeuten kann. Die erkannte Kurvencharakteristik hat den Vorteil, dass der Schlag bereits vor Erreichen einer hohen Beschleunigung erkannt werden kann. Weiters könnte ein Algorithmus eingesetzt werden, wodurch z.B. mittels Machine Learning aus den Beschleunigungsdaten frühzeitig ein Schlag erkannt werden kann. Die vorbestimmte Eigenschaft ist somit eine Eigenschaft, die in einem Algorithmus oder einer Machine-Learning-Datenbank hinterlegt sein kann.

Die Recheneinheit kann als einfacher integrierter Schaltkreis ausgeführt oder auch nur als simple Logikschaltung implementiert sein. Alternativ könnte es sich auch um einen Computer mit darauf gespeichertem Computerprogramm handeln. Besonders vorteilhaft ist, wenn die Recheneinheit und die zumindest zwei Sensoren auf einer gemeinsamen Platine verbaut sind, da dadurch nur ein einziges Element in oder an der Vorrichtung befestigt werden muss.

Die zwei Betriebsmodi der schaltbaren Komponente können auf unterschiedliche Art und Weise verwirklicht werden. In einer ersten Variante kann die schaltbare Komponente im ersten Betriebsmodus abgeschaltet sein. Sollte die schaltbare Komponente in dieser Variante eine zu langsame Reaktionszeit aufweisen, d.h. eine Zeit, bis die schaltbare Komponente nach dem Einschalten den ersten Messwert im zweiten Betriebsmodus liefert oder empfängt, kann der erste Betriebsmodus auch ein Standby-Modus sein. Unter einem Standby-Modus wird ein Betriebsmodus verstanden, bei dem die schaltbare Komponente im Vergleich zur abgeschalteten Variante strombeaufschlagt ist, aber keine Messwerte ausgibt oder empfängt, wodurch eine schnellere Reaktionszeit erzielt wird. Die meisten schaltbaren Komponenten weisen jedoch auch im ausgeschalteten Zustand eine ausreichende Reaktionszeit auf, sodass der Standby-Modus nicht vorgesehen werden muss.

Wenn die schaltbare Komponente bzw. eine der schaltbaren Komponenten der zweite Beschleunigungssensor oder ein Drucksensor ist, kann diese schaltbare Komponente im ersten Betriebsmodus eine geringere Rate an Messwerten pro Zeiteinheit als im zweiten Betriebsmodus liefern. Bei dieser Variante ist die Reaktionszeit äußerst kurz. Der erste Betriebsmodus wird als de-facto Standby-Modus eingesetzt und kann auch nur eine so geringe Rate aufweisen, dass eine Verfolgung der Schlagbewegung im ersten Betriebsmodus nicht möglich ist.

Es sei an dieser Stelle jedoch hervorgehoben, dass auch andere Varianten möglich sind. Es soll jedoch jeweils umgesetzt werden, dass der Stromverbrauch der schaltbaren Komponente erst dann steigt, wenn der jeweilige Event (bei Erreichen des Schwellwerts, der Kurvencharakteristik etc.) ausgelöst wird.

Aus den vorstehenden Ausführungen ist ersichtlich, dass die primäre Reduktion des Strombedarfs durch äußerst selektives Hinzuschalten der schaltbaren Komponente erfolgt. Eine sekundäre Reduktion des Strombedarfs kann dadurch erfolgen, dass der erste Beschleunigungssensor abgeschaltet oder in einen Betriebsmodus mit reduziertem Strombedarf versetzt werden kann, wenn sich die schaltbare Komponente im zweiten Betriebsmodus befindet. In einer Ausführungsform kann der erste Beschleunigungssensor auch selektiv abgeschaltet oder in einen Standby-Modus versetzt werden, wenn der zweite Beschleunigungssensor bereits Messwerte liefert.

Dem Schwellwert kommt bei der erfindungsgemäßen Lösung eine besondere Bedeutung zu, da er eine präzise Einstellung des Umschaltzeitpunkts vom ersten Betriebsmodus in den zweiten Betriebsmodus ermöglicht. Die Recheneinheit oder der erste Beschleunigungssensor könnte sogar einen Steuereingang aufweisen, über welchen der Schwellwert verstellt werden kann. Sollte sich beispielsweise herausstellen, dass die schaltbare Komponente eine zu lange Reaktionszeit aufweist (siehe oben), kann der Schwellwert gesenkt werden, da dies die Zeit verlängert, bis zu welcher der erste Beschleunigungssensor den von ihm maximal messbaren Beschleunigungsmesswert erreicht.

Im Allgemeinen ist bevorzugt, wenn sich der Schwellwert unterhalb eines maximal messbaren Beschleunigungsmesswertes des ersten Beschleunigungssensors befindet und nicht genau bei diesem. Beispielsweise kann der Schwellwert 50 %-95 % des maximal messbaren Beschleunigungsmesswertes des ersten Beschleunigungssensors betragen. Wenn der erste Beschleunigungssensor beispielsweise einen Messbereich von 16 g aufweist, kann der Schwellwert 8 g bis 15,2 g betragen. Es versteht sich, dass der Schwellwert je nach Anwendungsfall auch anders gewählt werden kann.

Ein Schwellwert unterhalb eines maximal messbaren Beschleunigungsmesswertes des ersten Beschleunigungssensors hat den Vorteil, dass eine gewisse Reaktionszeit des zweiten Sensors ermöglicht wird (siehe oben).

Wenn der zweite Sensor ein zweiter Beschleunigungssensor ist, wird vorgesehen, dass die beiden Beschleunigungssensoren unterschiedliche Messbereiche haben, d.h. der zweite Beschleunigungssensor kann dazu ausgebildet sein, höhere Beschleunigungsmesswerte als der erste Beschleunigungssensor zu messen. In diesem Fall kann der erste Beschleunigungssensor auch als Niedrigbeschleunigungssensor und der zweite Beschleunigungssensor als Hochbeschleunigungssensor bezeichnet werden. Der Niedrigbeschleunigungssensor kann einen Messbereich von bis zu 16 g in drei orthogonal zueinander stehenden Richtungen aufweisen und der Hochbeschleunigungssensor kann einen Messbereich von bis zu 64 g in drei orthogonal zueinander stehenden Richtungen aufweisen. Es wäre jedoch auch möglich, wenn zwei Beschleunigungssensoren eingesetzt werden, die denselben Messbereich aufweisen, aber z.B. unterschiedlich große interne Speicher oder unterschiedliche Sampling-Frequenzen aufweisen. Auch in diesem Fall ist die erfindungsgemäße Lösung vorteilhaft.

Weiters ist vorteilhaft, wenn der erste Beschleunigungssensor einen internen Speicher aufweist, der bevorzugt ein FIFO-Speicher ist. Dadurch können die im internen Speicher gespeicherten Daten ausgegeben werden, wenn die schaltbare Komponente vom ersten Betriebsmodus in den zweiten Betriebsmodus versetzt wird, um so die Messdaten zu erhalten, die kurz vor dem Aufprall aufgezeichnet wurden. Die Größe des Speichers kann derart ausgestaltet sein, dass Messdaten über einen vorbestimmten Zeitraum von z.B. 1 Sekunde gespeichert werden. Ältere Daten können hierbei automatisch überschrieben werden.

Besonders bevorzugt ist die Recheneinheit die schaltbare Komponente und ist im ersten Betriebsmodus abgeschaltet oder in einem Standby-Modus. Dies ist insbesondere mit den beiden vorgenannten Merkmalsgruppen kombinierbar, in welchem Fall die Recheneinheit dazu ausgebildet sein kann, nach dem Versetzen vom ersten Betriebsmodus in den zweiten Betriebsmodus die Daten des internen Speichers des ersten Beschleunigungssensors zu empfangen und beginnend ab dem Zeitpunkt des genannten Versetzens Beschleunigungsmesswerte des zweiten Beschleunigungssensors zu empfangen und zu speichern, bevorzugt über einen vorbestimmten Zeitraum oder bis ein Beschleunigungsschwellwert unterschritten wird. Danach liegen alle zu einem Schlag zugehörigen Mess-Rohdaten, auch die von weiteren Sensoren, wohlsortiert z.B. in einem Speicher der Recheneinheit vor. In weiterer Folge kann die Recheneinheit kontrollieren, ob es wirklich ein Schlag war (z.B. über einen Plausibilitätsprüfung), und die Schlagdauer schätzen, z.B. über einen Algorithmus oder Kurvenfunktionen. Das Schätzen der Schlagdauer ist deshalb von Vorteil, weil der Onboard-Speicher besser ausgenutzt werden kann. Auch die Übertragungsdauer der Messdaten zu einem weiteren Gerät kann hierdurch optimiert werden.

Wie eben erwähnt ist es daher auch vorteilhaft, wenn die Vorrichtung einen weiteren Speicher (auch Onboard-Speicher genannt) umfasst, in dem die Daten des internen Speichers des ersten Beschleunigungssensors und die vom zweiten Beschleunigungssensor empfangenen Daten sowie gegebenenfalls die von weiteren Sensoren ausgegebenen Messdaten verknüpft hinterlegt werden, wobei der Onboard-Speicher über eine externe Schnittstelle auslesbar ist. Der Onboard-Speicher kann flüchtig oder nichtflüchtig sein und die Messdaten über einen langen Zeitraum aufnehmen, bevorzugt über einen ganzen Wettkampftag. Außerdem eignet sich der Onboard-Speicher als Zwischenspeicher zur kabellosen Übertragung, z.B. über Bluetooth, wenn bei Übertragungsproblemen in einem Live-Modus (d.h. in einem Modus, in dem Messdaten sofort gesandt werden) Daten zu einem späteren Zeitpunkt erneut übertragen werden sollen.

Alternativ oder zusätzlich zur vorgenannten Variante, bei der die Recheneinheit die schaltbare Komponente ist, könnte auch der zweite Sensor die schaltbare Komponente bzw. eine der schaltbaren Komponenten sein. Hierbei ist der zweite Beschleunigungssensor bevorzugt im ersten Betriebsmodus abgeschaltet oder liefert im ersten Betriebsmodus eine geringere Rate an Beschleunigungsmesswerten als im zweiten Betriebsmodus. In diesen Varianten ist besonders bevorzugt, wenn sich der Schwellwert unterhalb eines maximal messbaren Beschleunigungsmesswertes des ersten Beschleunigungssensors befindet. Dadurch können beide Beschleunigungssensoren gleichzeitig über einen bestimmten Zeitraum Beschleunigungsmesswerte liefern oder eine Reaktionszeit des zweiten Beschleunigungssensors überbrückt werden.

Auch in der vorgenannten Variante kommt eine Recheneinheit zum Einsatz (die gegebenenfalls gleichzeitig mit dem zweiten Beschleunigungssensor in den zweiten Betriebsmodus versetzt wird), welche eine Schlagauswertung und/oder eine Speicherung der Messdaten in einem weiteren Speicher (Onboard-Speicher) vornehmen kann. Auch in dieser Variante kann der erste Beschleunigungssensor einen internen Speicher aufweisen, der von der Recheneinheit ausgelesen werden kann. Wenn die Recheneinheit ständig in Betrieb ist, kann diese auch ständig Messdaten des ersten Beschleunigungssensors empfangen, auswerten und speichern (wobei dies aufgrund der großen Datenmenge und des erhöhten Stromverbrauchs der Recheneinheit nicht immer vorteilhaft ist). In diesem Fall könnte die Recheneinheit dafür vorgesehen sein, eine Schlagerkennung durchzuführen, und nur dann, wenn es wahrscheinlich ist, dass ein Schlag vorliegt, den zweiten Sensor in den zweiten Betriebsmodus zu versetzen.

Im Allgemeinen kann daher davon gesprochen werden, dass die Vorrichtung einen Onboard-Speicher umfasst, in dem die Daten des internen Speichers des ersten Beschleunigungssensors und die (ab dem Versetzen in den zweiten Betriebsmodus) vom zweiten Beschleunigungssensor empfangenen Daten hinterlegt werden, wobei der Onboard-Speicher über eine externe Schnittstelle auslesbar ist. Es versteht sich, dass die externe Schnittstelle keine dedizierte Schnittstelle des Onboard-Speichers sein muss. Beispielsweise könnte die externe Schnittstelle ein mit der Recheneinheit verbundener Sendeempfänger (z.B. ein Bluetooth Sendeempfänger, insbesondere ein Bluetooth Low Energy, BLE, Sendeempfänger) sein, der mit der Recheneinheit verbunden ist, die wiederum Daten des Onboard-Speichers ausliest.

Weiters bevorzugt weist jener Beschleunigungssensor, der dazu ausgebildet ist, höhere Beschleunigungsmesswerte als der andere Beschleunigungssensor zu messen, eine höhere Samplingfrequenz auf (bevorzugt eine zumindest doppelt so hohe Samplingfrequenz) als der andere Beschleunigungssensor. Dies ist deshalb vorteilhaft, da der Hochbeschleunigungssensor die sich schnell ändernden und gleichzeitig hohen Beschleunigungsmesswerte bzw. negativen Beschleunigungswerte/Abbremswerte während eines Aufpralls messen soll und der Niedrigbeschleunigungssensor die durch den Athleten bzw. das bewegte Körperteil verursachten Beschleunigungsdaten, die dem Aufprall vorgelagert und verhältnismäßig niedrig sind, d.h. niedrig im Sinne der Änderungsrate und der Beschleunigungsamplitude messen soll. Durch diese reduzierte Samplingfrequenz kann eine weitere Energiereduktion erzielt werden.

Um die schaltbare Komponente wieder in den ersten Betriebsmodus zu überführen, sodass der Strombedarf wieder gesenkt wird, können unterschiedliche Varianten vorgesehen werden. Im einfachsten Fall können die Messwerte des ersten Beschleunigungssensors darauf hindeuten, dass die schaltbare Komponente nicht mehr benötigt wird, woraufhin diese wieder in den ersten Betriebsmodus versetzt werden kann.

Die Beschleunigungsmesswerte der beiden Beschleunigungssensoren dienen an sich unterschiedlichen Zwecken. Die Beschleunigungsmesswerte des Niedrigbeschleunigungssensors (in der Regel des ersten Beschleunigungssensors) dienen zur Verfolgung des Bewegungsablaufs, sodass z.B. eine Schlagtechnik und eine durchschnittliche sowie maximale Ausführungsgeschwindigkeit festgestellt werden kann.

Die Beschleunigungsmesswerte des Hochbeschleunigungssensors (in der Regel des zweiten Beschleunigungssensors) dienen jedoch einem anderen Zweck, nämlich der Bestimmung der Bremsbeschleunigung, Kontaktzeit, Schlagkraft und der Ermittlung des Ziels (z.B. Boxsack, Wand, Schlagpratzen, Körper - konkret kann man durch die Höhe der Negativbeschleunigung mittels statistischer Verfahren bzw. Machine Learning und gegebenenfalls in Verbindung mit einigen anderen Variablen feststellen, auf welche Art von Ziel geschlagen wird). Zu diesem Zweck kann die genannte Recheneinheit oder eine weitere Recheneinheit (wie z.B. ein Smartphone) dazu ausgebildet sein, aus Beschleunigungsmesswerten des zweiten Beschleunigungssensors (gegebenenfalls auch in Kombination mit Messdaten von weiteren Sensoren) während eines Aufpralls zusammen mit einer geschätzten oder vorab bestimmten effektiven Masse oder der Masse eines Ziels einen Kraftmesswert zu berechnen. Es ist ersichtlich, dass die Messwerte der beiden Beschleunigungssensoren auch unterschiedlichen Auswerteeinheiten zugeführt werden könnten, da diese unterschiedlichen Zwecken dienen.

Besonders bevorzugt umfasst die Vorrichtung eine inertiale Messeinheit, die einen der Beschleunigungssensoren (üblicherweise den Niedrigbeschleunigungssensor) und einen Drehratensensor umfasst, wobei die inertiale Messeinheit gesondert zum anderen Beschleunigungssensor vorliegt. In anderen Worten ist der andere Beschleunigungssensor nicht Teil der inertialen Messeinheit. Inertiale Messeinheiten umfassend Beschleunigungssensoren mit niedrigen Messbereichen wie bis zu 8 g oder bis zu 16 g sind kommerziell gut erhältlich. Wenn der Beschleunigungssensor der inertialen Messeinheit die schaltbare Komponente ist, kann die ganze inertiale Messeinheit die schaltbare Komponente darstellen. Alternativ könnte der Drehratensensor dauerhaft in Betrieb sein, während der Beschleunigungssensor der inertialen Messeinheit die schaltbare Komponente bildet.

In einer besonders bevorzugten Konfiguration ist der erste Beschleunigungssensor dazu ausgebildet, Beschleunigungsmesswerte von bis zu 16 g (oder bis zu 8 g) in drei orthogonal zueinanderstehenden Raumrichtungen zu messen, und/oder der zweite Beschleunigungssensor ist dazu ausgebildet, Beschleunigungsmesswerte von bis zu 64 g (oder bis zu 100 g oder bis zu 128 g) in drei orthogonal zueinanderstehenden Raumrichtungen zu messen. Insbesondere für den zweiten Beschleunigungssensor könnte aber auch vorgesehen werden, dass dieser Beschleunigungssensoren nur entlang einer Achse misst, und zwar der Normalschlagrichtung (d.h. in eine Richtung nach vorne, gesehen bezüglich des Schlaghandschuhes). Der zweite Beschleunigungssensor wird nämlich üblicherweise nicht zur Schlagnachverfolgung eingesetzt. Eine Messung der Beschleunigung in drei orthogonal zueinanderstehenden Raumrichtungen durch den zweiten Beschleunigungssensor ist jedoch vorteilhaft, um genauere Messergebnisse zu erhalten.

Alternativ könnte der zweite Sensor auch ein Drucksensor sein, in welchem Fall die Vorrichtung einen Schlaghandschuh mit einem fluidgefüllten Körper umfasst, in dem der Drucksensor vorliegt. Das Konzept der schaltbaren Komponente kann auch in diesem Fall umgesetzt werden, sodass entweder die Recheneinheit in den zweiten Betriebsmodus versetzt wird, wenn der erste Beschleunigungssensor oder der Drucksensor entsprechende Messdaten liefert; oder der Drucksensor könnte in den zweiten Betriebsmodus versetzt werden, wenn der erste Beschleunigungssensor entsprechende Daten liefert.

Vorteilhafte und nicht einschränkende Ausführungsformen der in den Ansprüchen wiedergegebenen Erfindung werden nachfolgend anhand der Zeichnungen näher erläutert.
Figur 1 zeigt eine Vorrichtung zur Bestimmung einer Schlageigenschaft in einer schematischen Ansicht mit den im Inneren der Vorrichtung befindlichen Komponenten (in einer Variante ohne Drucksensor).
Figur 2a zeigt die Vorrichtung von Figur 1 in einer schematischen Perspektivansicht mit darin befindlichen Komponenten.
Figur 2b zeigt ein schematisches Blockdiagramm von ausgewählten Komponenten der Vorrichtung.
Die Figuren 3, 4 und 5 zeigen die bei einem Aufprall auftretenden Beschleunigungskurven bei einer Vorrichtung entlang einer x-Achse (Figur 3), einer y-Achse (Figur 4) und einer z-Achse (Figur 5).
Figur 6 zeigt drei überlagerte Beschleunigungskurven und die Komponenten zum Bestimmen der jeweiligen Beschleunigungsmesswerte.
Figur 7 zeigt die von einem ersten Beschleunigungssensor gemessenen Beschleunigungsmesswerte.
Figur 8 zeigt die von einem zweiten Beschleunigungssensor gemessenen Beschleunigungsmesswerte.
Figur 9 zeigt eine Ausführungsform, bei der ein Drucksensor in einem Schlaghandschuh vorgesehen ist.

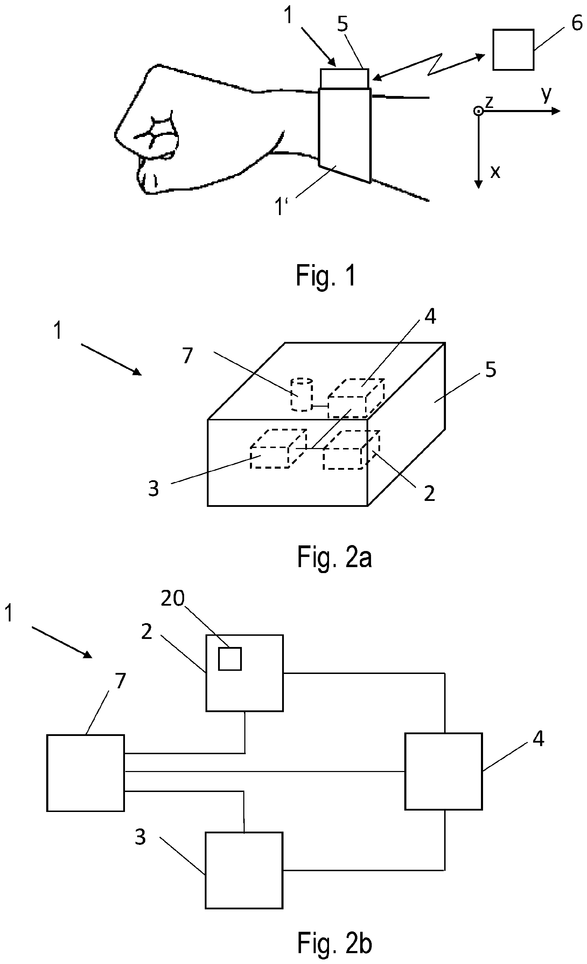

Figur 1 zeigt eine Vorrichtung 1, die zur Bestimmung einer Schlageigenschaft wie einer Schlagbeschleunigung, Schlaggeschwindigkeit, Schlagkraft oder Schlagtechnik dient. Die Vorrichtung 1 kann beispielsweise eine Box mit Kunststoffgehäuse 5 sein, in der die unten beschriebene Elektronik vorliegt. Die Box ist derart klein ausgeführt, dass diese auf einem Körperteil, insbesondere der Hand, befestigbar ist, z.B. mit den Maßen 4 cm x 3 cm x 1 cm.

Im dargestellten Beispiel wird eine Bandage 1' am Handgelenk befestigt, welches eine Aufnahme für die Vorrichtung 1 aufweist. Die Vorrichtung 1 wird beispielsweise in eine Tasche der Bandage 1' eingeschoben, und die Bandage 1' wird daraufhin um das Handgelenk gewickelt. In der gleichen Art und Weise könnte die Vorrichtung 1 auch am Fuß bzw. Fußgelenk befestigt werden. Es versteht sich, dass die Vorrichtung 1 auch unmittelbar auf dem Körper (oder z.B. auch einem Crash-Test-Dummy) befestigbar ist.

Die oben beschriebene Vorrichtung 1 bzw. Box könnte auch in eine Tasche eines Schlaghandschuhes oder eines Schützers wie eines Fußschützers eingeführt werden, sodass keine Bandage 1' benötigt wird. Die Vorrichtung 1 könnte auch einen Schlaghandschuh umfassen oder ein anderes Schlagelement selbst sein, in dem die unten beschriebene Elektronik untergebracht ist. Weiters könnte die Vorrichtung ein anderes Körperschutzteil wie einen Kopfschutz umfassen, in dem die im Folgenden beschriebene Elektronik aufgenommen ist und "passiv" den Aufprall misst. Die Vorrichtung könnte auch auf einem Boxsack montiert sein.

Wie in den Figuren 2a und 2b dargestellt ist, umfasst die Vorrichtung 1 in einer ersten Ausführungsform einen ersten Beschleunigungssensor 2 und einen zweiten Beschleunigungssensor 3. Die beiden Beschleunigungssensoren 2, 3 unterscheiden sich üblicherweise voneinander hinsichtlich ihres Messbereichs, wobei in dieser Ausführungsform der zweite Beschleunigungssensor 3 höhere Beschleunigungsmesswerte als der erste Beschleunigungssensor 2 messen kann. In anderen Worten hat der erste Beschleunigungssensor 2 einen kleineren Messbereich als der zweite Beschleunigungssensor 3. Beide Beschleunigungssensoren 2, 3 können dieselbe oder unterschiedliche Abtastraten (Beschleunigungsmesswerte pro Zeiteinheit) aufweisen, die sich entsprechend des Betriebszustandes gegebenenfalls auch ändern können.

Weiters umfasst die Vorrichtung 1 eine Recheneinheit 4, die mit den beiden Beschleunigungssensoren 2, 3 verbunden ist. In der dargestellten Ausführungsform befindet sich die Recheneinheit 4 unmittelbar neben den genannten Beschleunigungssensoren 2, 3, sodass die Beschleunigungssensoren 2, 3 mittels Kabeln oder anderen Verbindungen auf einer Platine mit der Recheneinheit 4 verbunden sein können. Die Recheneinheit kann z.B. eine einfache Anordnung von Entscheidern (Logikschaltung) oder ein komplexes System (Mikrokontroller) sein.

Weiters kann auch eine weitere Auswerteeinheit 6 vorgesehen sein (die gegebenenfalls mit der Recheneinheit 4 über Funk kommunizieren kann), welche die Beschleunigungsdaten und/oder die aus den Beschleunigungsdaten bestimmte Schlageigenschaft, insbesondere eine Schlagbeschleunigung, Schlaggeschwindigkeit oder Schlagkraft, berechnet und/oder speichert. Dies kann z.B. für ein Offline-Training und Data-looping eingesetzt werden. Die Auswerteeinheit kann sich innerhalb oder außerhalb der Vorrichtung befinden.

Weiters befindet sich eine Batterie 7 in der Vorrichtung 1 oder ist mit dieser verbunden. Die Batterie 7 ist mit beiden Beschleunigungssensoren 2, 3 und mit der Recheneinheit 4 verbunden. Es ist einleuchtend, dass die Batterie 7 einerseits leicht und klein genug ausgeführt werden soll, um keine Behinderung für einen Benutzer der Vorrichtung 1 darzustellen, aber andererseits eine möglichst lange Laufzeit aufweisen soll, damit die Vorrichtung 1 möglichst lange ohne Nachladen oder Austausch der Batterie benutzt werden kann.

In einer bevorzugten Variante kann zur Umsetzung des genannten Aufbaus eine Elektronikplatine im Inneren der Vorrichtung 1 vorgesehen sein, auf der sich neben den genannten Beschleunigungssensoren 2, 3 auch weitere Komponenten befinden könnten, z.B. eine drahtlose Übertragungseinheit (z.B. zur Kommunikation der Recheneinheit 4 mit der externen Auswerteeinheit 6). Weiters befindet sich die Batterie 7 in der Vorrichtung 1, welche mit der Platine und damit mit den auf der Platine befindlichen Komponenten verbunden ist. Es versteht sich, dass auch weitere Ausführungsformen möglich sind, z.B. wenn die Vorrichtung 1 einen Schlaghandschuh umfasst und eine Batterie 7 lose auf dem Schlaghandschuh befestigbar ist.

Wie bereits oben erwähnt ist bevorzugt, wenn sich die beiden Beschleunigungssensoren 2, 3 und die Recheneinheit 4 (besonders bevorzugt auf der gemeinsamen Platine) und gegebenenfalls auch die Batterie innerhalb eines Kunststoffgehäuses 5 befinden, das die Form einer Box aufweisen kann.

Sowohl der erste Beschleunigungssensor 2 als auch der zweite Beschleunigungssensor 3 sind in der Regel dazu ausgebildet, Beschleunigungsmesswerte in drei orthogonal zueinanderstehenden Raumrichtungen x, y, z aufzuzeichnen. Üblicherweise sind die Raumrichtungen x, y, z der Beschleunigungssensoren 2, 3 ausgerichtet, d.h. eine der Raumrichtungen y zeigt an der Vorrichtung 1 nach vorne (in Normalschlagrichtung), eine der Raumrichtungen x zeigt nach unten (d.h. liegt normal und vertikal zur Normalschlagrichtung) und die dritte Achse z verläuft orthogonal zu den anderen Raumrichtungen x, y (d.h. liegt normal und horizontal zur Normalschlagrichtung), sodass jeweils zugehörige Beschleunigungsmesswerte ax, ay, az gemessen werden können. In anderen Ausführungsformen könnte einer oder beide der Beschleunigungssensoren 2, 3 aber auch nur die Beschleunigung entlang einer Achse messen, beispielsweise, wenn diese Achse y entlang der Normalschlagrichtung der Vorrichtung 1 ausgerichtet ist. Bereits an dieser Stelle sei festgehalten, dass es nicht weiter relevant ist, mit welchem Vorzeichen die Beschleunigung aufgezeichnet wird, d.h. ob eine Negativbeschleunigung oder eine Positivbeschleunigung gemessen wird. Dies trifft auch für die Terminologie "Schwellwert überschreiten" und "Schwellwert unterschreiten" zu, da diesbezüglich von einem Absolutwert ausgegangen wird und es irrelevant ist, ob die Werte in der negativen oder positiven Domäne gemessen werden. Dennoch könnten in eine +x/+y/+z Richtung ein anderer Schwellwert als in die jeweils entgegengesetzte -x/-y/-z Richtung vorgesehen sein, auch die Schwellwerte der genannten 6 einzelnen Richtungen x, y und z können individuell gewählt werden.

Zweck der Vorrichtung 1 ist es, die bei einem Schlag auftretende Beschleunigung aufzuzeichnen. Dies umfasst üblicherweise einen Zeitraum vor dem Aufprall des Schlages und einen Zeitraum nach dem Aufprall des Schlages, d.h. der Begriff "bei einem Aufprall/Schlag" oder "während einem Aufprall/Schlag" umfasst einen Zeitraum, der um den Aufprall herum liegt. Insbesondere soll die Anlaufbeschleunigung als auch die bei einem Aufprall auftretende Bremsbeschleunigung gemessen werden.

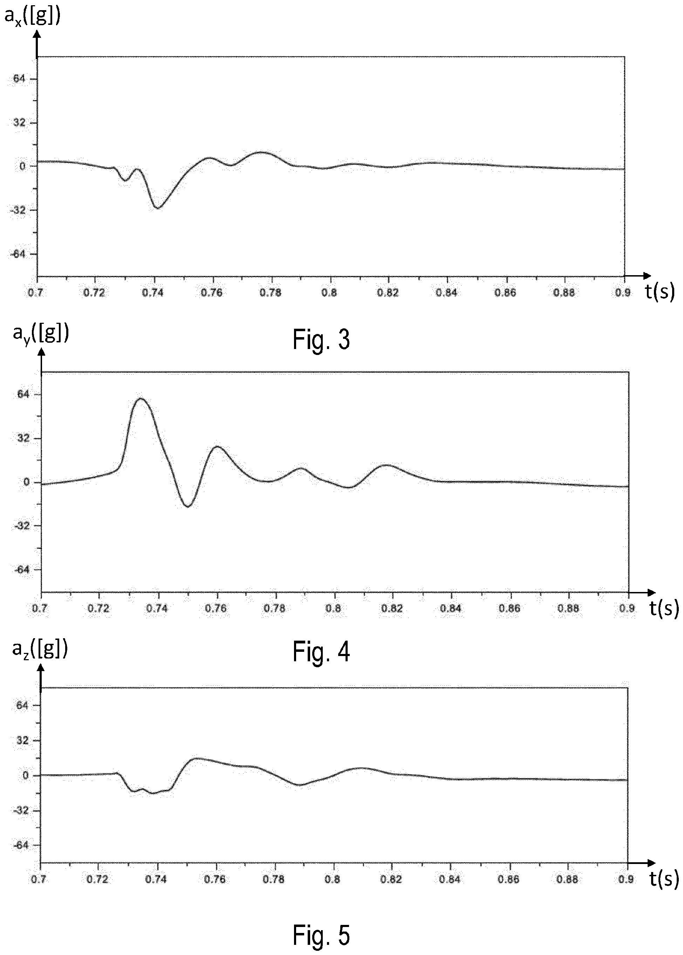

In diesem Zusammenhang zeigen die Figuren 3 bis 5 die auftretenden Beschleunigungswerte ax, ay, az bei einem Schlag mit der Vorrichtung 1, wobei die Schlagkraft Fs=2,5 kN betrug und das Ziel ein Sandsack war. Auf der vertikalen Achse ist die Beschleunigung in Einheiten von g eingetragen, wobei g=9,81m/s². Auf der horizontalen Achse ist ein Zeitverlauf eingetragen, wobei der Aufprall des Schlags bei ca. 0,72 Sekunden beginnt. Bei dem Schlag, für den die Beschleunigungswerte dargestellt sind, traf die Vorrichtung 1 auf eine ebene Fläche. Der Beschleunigungswert ay wurde entlang der Schlagrichtung gemessen und die Beschleunigungswerte ax, az in orthogonalen Richtungen normal zur Schlagrichtung, siehe auch das Koordinatensystem in Figur 1.

Es ist nun die Aufgabe der Erfindung, diesen Beschleunigungsverlauf aus den Figuren 3 bis 5 (oder zumindest den Beschleunigungsverlauf in Normalschlagrichtung) möglichst energieeffizient mit der oben beschriebenen Vorrichtung 1 aufzuzeichnen. Hierzu können verschiedene Ausführungsvarianten eingesetzt werden, die jeweils gemeinsam haben, dass eine Komponente als schaltbare Komponente ausgeführt ist und erst bei der Detektion eines (potentiellen) Schlages angeschaltet bzw. mit einem erhöhtem Strombedarf betrieben wird. Im Allgemeinen wird davon gesprochen, dass die schaltbare Komponente von einem ersten Betriebsmodus in einen zweiten Betriebsmodus gebracht wird, wobei die schaltbare Komponente im ersten Betriebsmodus einen geringeren Strombedarf aufweist als im zweiten Betriebsmodus.

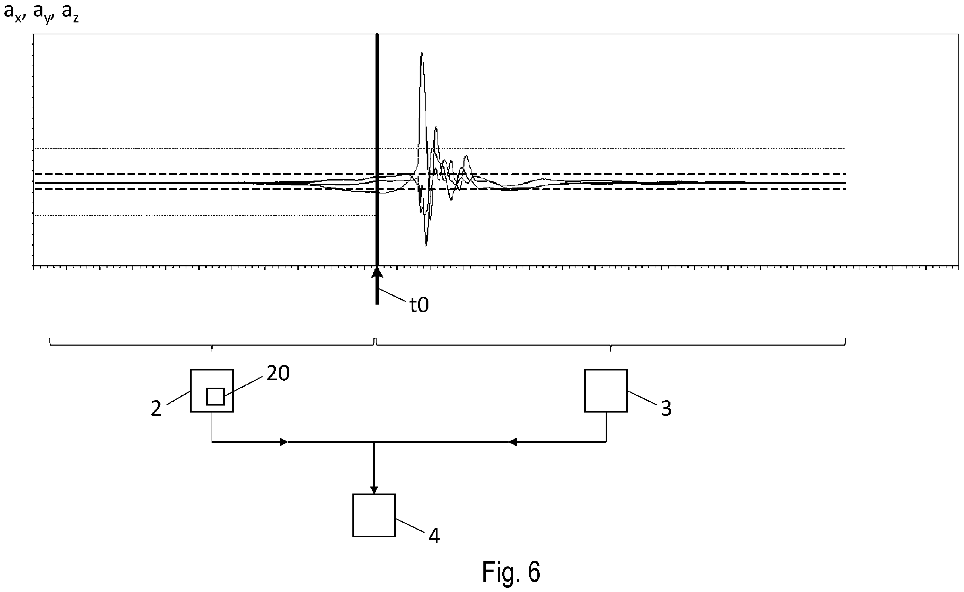

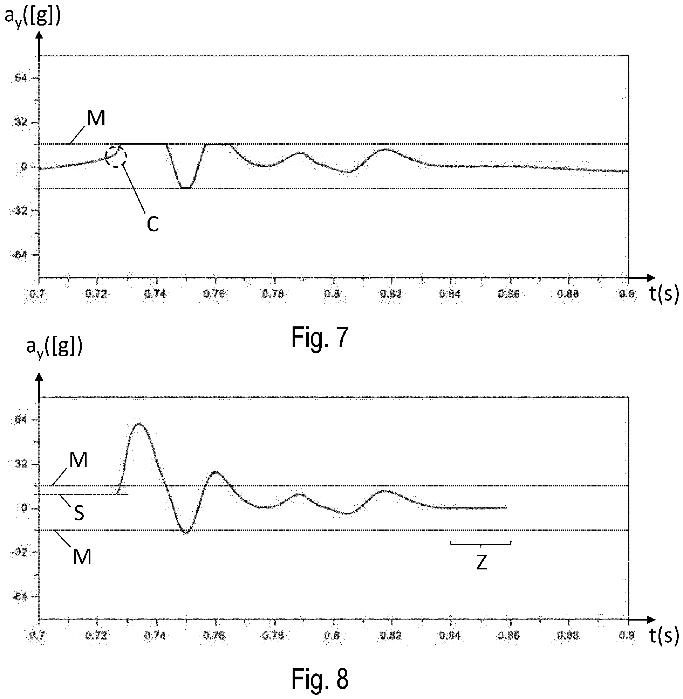

In der ersten Variante sind der erste Beschleunigungssensor 2 und der zweite Beschleunigungssensor 3 dauerhaft in Betrieb. Die Recheneinheit 4 ist in dieser Ausführungsform die schaltbare Komponente und ruft im ersten Betriebsmodus keine Messdaten von den Sensoren ab. Wechselt die Recheneinheit 4 in den zweiten Betriebsmodus, beginnt diese Messdaten auszulesen. Der erste Beschleunigungssensor 2 weist einen niedrigeren Messbereich als der zweite Beschleunigungssensor 3 auf. In dieser Variante soll der erste Beschleunigungssensor 2 die Messdaten "vor" dem Aufprall aufzeichnen (siehe z.B. Figur 7) und der zweite Beschleunigungssensor 3 die Messdaten "nach" dem Aufprall erfassen. Die Recheneinheit soll erst dann hinzugeschalten werden, wenn der Aufprall (üblicherweise durch den zweiten Beschleunigungssensor 3) zum Zeitpunkt t0 erkannt wird. Figur 6 zeigt eine hierfür vorgesehene Variante, wobei in diesem Diagramm die drei orthogonal zueinander gemessenen Beschleunigungsmesswerte ax, ay, az, gemessen über die Zeit t, überlagert dargestellt sind.

Hierzu umfasst der erste Beschleunigungssensor 2 einen internen Speicher 20, um ständig Daten aufzuzeichnen. Beispielsweise können die Beschleunigungsdaten eines vorbestimmten Zeitraumes aufgezeichnet werden, z.B. die jeweils x letzten Sekunden, beispielsweise, die jeweils letzten 640 ms, was einem Speicherbedarf von 64 kB entspricht. Der interne Speicher 20 kann beispielsweise als FIFO (First In First Out) ausgestaltet sein, wodurch leicht ermöglicht wird, dass immer die jeweils x letzten Sekunden oder Minuten aufgezeichnet werden. Nicht dargestellt ist, dass auch der zweite Beschleunigungssensor 3 einen internen Speicher aufweisen kann, in dem jedoch üblicherweise Messdaten über einen geringeren Zeitraum speicherbar sind, als dies beim vorgenannten internen Speicher 20 der Fall ist.

Der zweite Beschleunigungssensor 3, der auch als Hochbeschleunigungssensor bezeichnet werden kann, hat hingegen besondere Triggermöglichkeiten, sodass dieser dazu eingesetzt wird, den Beginn eines Schlages zu erkennen und die Recheneinheit 4 vom schlafenden Zustand in einen wachen Zustand zu versetzen, d.h. vom ersten Betriebsmodus in den zweiten Betriebsmodus zu bringen. Die verbesserten Triggermöglichkeiten des zweiten Beschleunigungssensor 3 ergeben sich z.B. durch den größeren Messbereich und bevorzugt auch durch eine größere Samplingfrequenz, d.h. Ausgaberate von Beschleunigungsmesswerten des zweiten Beschleunigungssensors 3 im Vergleich mit dem ersten Beschleunigungssensor 2.

Sobald der zweite Beschleunigungssensor 3 den Beginn t0 eines Aufpralls erkennt, z.B. indem die von diesem gemessenen Beschleunigungsmesswerte einen Schwellwert S überschreiten, kann dieser die Recheneinheit 4 aufwecken, z.B. indem ein Interrupt-Signal (im Allgemeinen ein Signal) an diese gesandt wird. Dadurch wird die Recheneinheit 4 in den zweiten Betriebsmodus versetzt. Davor war der Strombedarf der Recheneinheit nur gering oder sogar nicht vorhanden. Sobald die Recheneinheit 4 aufgeweckt ist, empfängt diese die Daten des internen Speichers 20 des ersten Beschleunigungssensors, d.h. Beschleunigungsmesswerte über die "Vergangenheit" vor dem Beginn des Aufpralls. Auch der interne Speicher des zweiten Beschleunigungssensors 3 ist von der Recheneinheit 4 auslesbar, z.B. um eine Reaktionszeit der Recheneinheit 4 zu überbrücken.

Weiters empfängt die Recheneinheit 4 Beschleunigungsmesswerte vom zweiten Beschleunigungssensor 3 ab dem Zeitpunkt t0, an dem die Recheneinheit 4 aufgeweckt worden ist, d.h. in den zweiten Betriebsmodus versetzt worden ist. Es ist ersichtlich, dass die Recheneinheit 4 dadurch Beschleunigungsmesswerte der zu t0 relativen Vergangenheit vom ersten Beschleunigungssensor 2 empfängt und Beschleunigungsmesswerte der zu t0 relativen Zukunft vom zweiten Beschleunigungssensor 3.

Es sei festgehalten, dass auch der zweite Beschleunigungssensor 3 einen internen Speicher aufweisen könnte, jedoch ist dies in der vorliegenden Ausführungsform nicht notwendig. Wenn sich die Recheneinheit 4 im ersten Betriebsmodus befindet, d.h. schläft oder ausgeschaltet ist, sind beide Beschleunigungssensoren 2, 3 aktiv und buffern intern Messdaten, ohne dass die Recheneinheit 4 diese abruft.

Wie bereits erwähnt liest die Recheneinheit 4 Beschleunigungsmesswerte vom zweiten Beschleunigungssensor 3 aus, wenn sich die Recheneinheit 4 im zweiten Betriebsmodus befindet. Vom Beschleunigungssensor 2 muss die Recheneinheit 4 keine "aktuellen" Beschleunigungsmesswerte lesen und macht dies üblicherweise auch nicht. Bevorzugt ist jedoch, wenn der erste Beschleunigungssensor 2 Teil einer IMU ist, die auch einen Drehratensensor umfasst. Die aktuellen Messdaten des Drehratensensors, die nach dem Versetzen in den zweiten Betriebsmodus von diesem gemessen werden, könnten auch nach dem Aufwecken der Recheneinheit 4 von dieser gelesen werden. Der Drehratensensor könnte auch Daten im internen Speicher 20 abspeichern, die zusammen mit den historischen Daten des ersten Beschleunigungssensors 2 an die Recheneinheit 4 gesandt werden.

Wie oben bereits erläutert wurde kann die Recheneinheit 4 aufgeweckt werden, wenn die Beschleunigungsmesswerte des zweiten Beschleunigungssensors 3 einen Schwellwert überschreiten. In anderen Varianten könnte das Aufwecksignal auch ausgegeben werden, wenn ein Schwellwert über einen vorbestimmten Zeitraum überschritten wird, z.B. wenn 3 g über 20 ms überschritten werden. Alternativ könnte der zweite Beschleunigungssensor 3 auch in den zweiten Betriebsmodus wechseln, wenn eine vorbestimmte Kurvencharakteristik C erkannt wird, wie sie beispielsweise in Figur 7 ersichtlich ist. Es ist zu erkennen, dass an dieser Stelle der Schlag beginnt, da die Beschleunigungsmesswerte sprunghaft ansteigen. Die Stärke des sprunghaften Anstiegs kann durch die Kurvencharakteristik definiert sein. Um noch frühere Erkennungen zu ermöglichen, wann der zweite Beschleunigungssensor 3 in den zweiten Betriebsmodus wechseln soll, kann auch ein Algorithmus, insbesondere ein Machine-Learning-Algorithmus eingesetzt werden, der eine vorbestimmte Eigenschaft der Beschleunigungsmesswerte erkennt.

In einer zweiten Variante mit schaltbarer Komponente kann einer der beiden Beschleunigungssensoren 2, 3 von einem ersten Betriebsmodus in einen zweiten Betriebsmodus versetzt werden. Dies ist insbesondere dann vorteilhaft, wenn der erste Beschleunigungssensor 2 in der Regel dauerhaft betrieben wird und der zweite Beschleunigungssensor 3, der einen höheren Messbereich als der erste Beschleunigungssensor 2 aufweist, die schaltbare Komponente darstellt.

In dieser Variante ist der erste Betriebsmodus im einfachsten Fall ein ausgeschalteter Zustand und der zweite Betriebsmodus ein eingeschalteter Zustand, bei dem der zweite Beschleunigungssensor 3 Beschleunigungsmesswerte erfasst oder zumindest in einem internen Speicher buffert. In einer weiteren Variante könnte der zweite Beschleunigungssensor 3 im ersten Betriebsmodus auch Beschleunigungsmesswerte mit einer ersten Rate aufnehmen und im zweiten Betriebsmodus mit einer zweiten Rate erfassen, wobei die erste Rate geringer ist als die zweite Rate. Die zweite Rate ist insbesondere eine maximale Rate an Beschleunigungsmesswerten pro Zeiteinheit, z.B. 1000 Hz. Die erste Rate ist ein vorbestimmter Prozentsatz davon, z.B. 10 %, in welchem Fall die erste Rate 100 Hz betragen würde. Es versteht sich, dass auch andere Raten gewählt werden können.

In jenem konkreten Beispiel, das nun anhand der Figuren 7 und 8 beschrieben wird, war der erste Beschleunigungssensor 2 dazu ausgebildet, Beschleunigungen von bis zu 16 g zu messen, was in den Figuren 7 und 8 durch den Messbereich M dargestellt ist, und der zweite Beschleunigungssensor 3 war dazu ausgebildet, Beschleunigungen von bis zu 64 g zu messen.

In Figur 7 ist dargestellt, welche Messwerte der erste Beschleunigungssensor 2 ausgibt, wenn ein Beschleunigungsverlauf wie in Figur 4 dargestellt vorliegt. Es werden alle Messwerte korrekt ausgegeben, die innerhalb des Messbereichs M von 16 g liegen. Sobald die Beschleunigung jedoch 16 g überschreitet, ist dies durch den ersten Beschleunigungssensor 2 nicht mehr messbar, sodass dieser entweder weiterhin den maximalen Beschleunigungsmesswert von 16 g ausgibt (obwohl der tatsächliche Beschleunigungswert darüber liegt), oder es wird ein Fehlersignal ausgegeben.

Die Recheneinheit 4 (die in dieser Variante entweder dauerhaft betrieben wird oder erst angeschaltet wird, wenn der erste Beschleunigungssensor eine Anlaufbeschleunigung für einen späteren Aufprall erkennt) kann den ersten Beschleunigungssensor 2 entweder derart ansteuern, dass dieser über die gesamte Messdauer angeschaltet ist, oder kann diesen abschalten (oder dessen Leistung reduzieren), wenn sich der zweite Beschleunigungssensor 3 im zweiten Betriebsmodus befindet.

Die Recheneinheit 4 (oder der erste Beschleunigungssensor 2) steuert den zweiten Beschleunigungssensor 3 nun derart an, dass dieser nicht die ganze Zeit eingeschaltet ist oder mit voller Leistung läuft (d.h. dass sich der zweite Beschleunigungssensor 3 nicht die ganze Zeit im ersten Betriebsmodus befindet), sondern der zweite Beschleunigungssensor 3 wird nur selektiv in den zweiten Betriebsmodus versetzt, wie in Figur 8 ersichtlich ist.

Konkret wird der zweite Beschleunigungssensor 3 dann in den zweiten Betriebsmodus versetzt, wenn die Beschleunigungsmesswerte des ersten Beschleunigungssensors 2 einen Schwellwert S erreichen. Der Schwellwert S liegt in der Regel innerhalb des Messbereichs M des ersten Beschleunigungssensors 2, d.h. unterhalb des Absolutwerts der maximal messbaren Beschleunigung des ersten Beschleunigungssensors 2. Im Beispiel der Figur 8 ist ersichtlich, dass der erste Schwellwert S bei 14 g lag, d.h. sobald der erste Beschleunigungssensor 2 erstmals 14 g gemessen hat, wurde der zweite Beschleunigungssensor 3 in den zweiten Betriebsmodus versetzt (im dargestellten Beispiel lieferte der zweite Beschleunigungssensor 3 sofort Messwerte, wobei dies nicht zwingend ist; es könnte sogar eine gewisse Messwertlücke vorliegen, die bevorzugt vor einem Maximalwert endet). Davor befand sich der zweite Beschleunigungssensor 3 im ersten Betriebsmodus, d.h. war ausgeschalten oder mit einer reduzierten Leistung betrieben. In anderen Fällen könnte der Schwellwert S aber auch mit der Grenze des Messbereichs M, d.h. mit dem Absolutwert der maximal messbaren Beschleunigung des ersten Beschleunigungssensors 2 zusammenfallen, sodass der Schwellwert S in diesem Beispiel bei 16 g liegen würde. Ein Schwellwert S, der innerhalb des Messbereichs M des ersten Beschleunigungssensors 2 liegt, hat jedoch unter anderem den Vorteil, dass sich die Messreihen der beiden Beschleunigungssensoren 2, 3 überlappen können, wodurch diese einfacher miteinander korreliert werden können.

In Figur 8 ist dargestellt, dass der zweite Beschleunigungssensor 3 beim Schwellwert S (hier 14 g) eingeschaltet wurde. Dies ermöglicht, dass der zweite Beschleunigungssensor 3 insbesondere jenen Bereich von 16 g bis 64 g (bzw. -16 g bis -64 g) genau aufzeichnen kann, der außerhalb des Messbereichs M des ersten Beschleunigungssensors 2 liegt. Es ist aber auch ersichtlich, dass der zweite Beschleunigungssensor 3 nicht wieder sofort abgeschaltet werden muss, wenn der Schwellwert S wieder unterschritten wird, sondern auch noch einige Zeit danach weitermessen kann. Dies ist insbesondere deshalb relevant, da erwartet wird, dass bei einem Schlag nach einem ersten Maximalwert weitere (positive und negative) Maximalwerte auftreten.

In der Variante von Figur 8 wird der zweite Beschleunigungssensor 3 wieder in den ersten Betriebsmodus versetzt, wenn der zweite Beschleunigungssensor 3 über ein vorbestimmtes Zeitfenster Z nur eine sich leicht ändernde Beschleunigung misst. Alternativ könnte der zweite Beschleunigungssensor 3 wieder in den ersten Betriebsmodus versetzt werden, nachdem ein Schwellwert R (der anders als der Schwellwert S gewählt werden kann) für eine vorbestimmte Zeit, z.B. 1 Sekunde, 2 Sekunden oder auch 10 Sekunden, nicht überschritten wurde. In einer weiteren Variante könnte der zweite Beschleunigungssensor 3 nach einer vorbestimmten Zeit nach dem Versetzen vom ersten in den zweiten Betriebsmodus wieder in den ersten Betriebsmodus wechseln, z.B. nach 1 Sekunde, 2 Sekunden oder auch 10 Sekunden. In einer weiteren Ausführungsform könnte der zweite Beschleunigungssensor 3 wieder in den ersten Betriebsmodus versetzt werden, wenn der Schwellwert S (oder ein anderer Schwellwert innerhalb des Messbereichs M) wieder unterschritten wird.

In den Beispielen oben hatte der erste Beschleunigungssensor 2 einen Messbereich M von 16 g und der zweite Beschleunigungssensor 2 einen Messbereich von 64 g (an dieser Stelle sei festgehalten, dass unter dem Begriff "Messbereich von x g" verstanden wird, dass Beschleunigungsmesswerte von -x g bis +x g messbar sind). Es versteht sich jedoch, dass die Beschleunigungssensoren 2, 3 auch andere Messbereiche aufweisen könnten, z.B. könnte der erste Beschleunigungssensor 2 einen Messbereich von 8 g bis 32 g aufweisen und der zweite Beschleunigungssensor 3 könnte einen Messbereich von mehr als 16 g oder mehr als 32 g aufweisen, z.B. einen Messbereich von im Wesentlichen 32 g oder 128 g.

Auch in dieser Ausführungsform kann der erste Beschleunigungssensor 2 Teil einer inertialen Messeinheit (IMU, inertial measurement unit) sein, die neben den Beschleunigungsmesswerten auch Drehraten, bevorzugt um drei im Wesentlichen orthogonale Raumrichtungen, bestimmten kann. Die Drehraten werden bevorzugt auch dann ausgegeben, wenn sich der zweite Beschleunigungssensor 3 im zweiten Betriebsmodus befindet. Dadurch kann eine genaue Verfolgung des Bewegungsablaufs ermöglicht werden und damit die Schlagart allein aus den Daten dieser inertialen Messeinheit bestimmt werden. Der erste Beschleunigungssensor 2 ist somit beispielsweise dauerhaft im Betrieb und verfolgt den Schlag an sich und der zweite Beschleunigungssensor 3 wird nur selektiv hinzugeschalten, wenn der Schwellwert erreicht wird, um das genaue Aufprallverhalten der Vorrichtung 1 zu bestimmen.

Die vom Beschleunigungssensor 2, 3 mit dem höheren Messbereich gemessenen Beschleunigungsmesswerte können insbesondere dazu eingesetzt werden, um einen Kraftmesswert (Schlagkraft) zu berechnen, was durch die genannte Recheneinheit 4 oder eine weitere Recheneinheit (die sich z.B. außerhalb der Vorrichtung 1 befindet und z.B. ein stationärer Rechner ist) durchgeführt werden kann. Um die Schlagkraft F zu berechnen, können die während eines Aufpralls der Vorrichtung 1 gemessenen Beschleunigungsmesswerte a mit einer geschätzten oder vorab bestimmten effektiven Masse m multipliziert werden. Die Schlagkraft F berechnet sich sodass aus F=m*a, wobei a die gemessene (Spitzen-)Beschleunigung und m die genannte effektive Masse ist. Auch ein Impuls p kann ermittelt werden als p= fF*dt.

Auch eine Schlaggeschwindigkeit v kann ermittelt werden, indem die gemessene Beschleunigung a integriert wird. Aus der Schlaggeschwindigkeit v kann wiederum eine kinetische Energie Ekin des Schlages berechnet werden als Ekin=m*v²/2. Weiters kann eine Schlaglänge ermittelt werden, die gegebenenfalls auch mit den oben genannten Schlageigenschaften verknüpft werden kann. Diese Berechnungen können für alle hierin beschriebenen Ausführungsformen herangezogen werden.

Üblicherweise werden die vom ersten Beschleunigungssensor 2 gemessenen Beschleunigungen dazu herangezogen, um eine Schlagart oder Schlaggeschwindigkeit zu berechnen und die vom zweiten Beschleunigungssensor 3 gemessenen Beschleunigungen werden dazu herangezogen, um eine Schlagkraft zu berechnen. Diese Berechnungen können durch die Recheneinheit 4 oder eine externe Recheneinheit erfolgen.

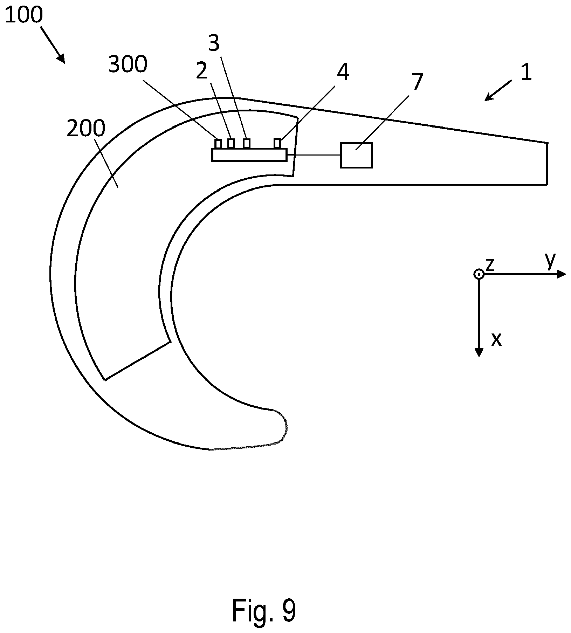

In einer weiteren Variante kann die Vorrichtung zusätzlich oder alternativ zum zweiten Beschleunigungssensor einen Drucksensor 300 umfassen, wie schematisch in Figur 9 dargestellt ist. Figur 9 zeigt einen Schlaghandschuh 100, der einen fluidgefüllten Körper 200 umfasst. Der fluidgefüllte Körper 200 besteht aus einer verformbaren Hülle, die mit einem Fluid, insbesondere einem Gas wie Luft oder auch einer Flüssigkeit, gefüllt ist. Im Inneren des fluidgefüllten Körpers 200 befindet sich zudem ein Drucksensor 300, der den hydrostatischen Druck innerhalb des fluidgefüllten Körpers 200 misst und an die Recheneinheit 4 weiterleitet, die sich innerhalb oder außerhalb des Schlaghandschuhes 1 befinden kann. Die Recheneinheit 4 kann vom Drucksensor 300 gemessene Druckmesswerte in Kraftmesswerte umrechnen, sodass die Schlagkraft des Schlaghandschuhes 1 ermittelbar ist. Weiters umfasst diese Ausführungsvariante (zumindest) den ersten Beschleunigungssensor 2.

Die Ausführungsform von Figur 9 kann entweder analog zur ersten vorgenannten Variante oder zur zweiten vorgenannten Variante betrieben werden. In anderen Worten können erstens der erste Beschleunigungssensor 2 und der Drucksensor 300 dauerhaft betrieben werden. Sobald der erste Beschleunigungssensor 2 den Beginn eines Aufpralls erkennt, wie zuvor über einen Schwellwertvergleich, eine Kurvencharakteristik etc., wird die Recheneinheit 4 vom ersten Betriebsmodus in den zweiten Betriebsmodus versetzt. Sobald sich die Recheneinheit 4 im zweiten Betriebsmodus befindet, liest diese den internen Speicher 20 des ersten Beschleunigungssensors 2 aus und ermittelt danach zyklisch die Messdaten des Drucksensors, um hieraus die Schlagkraft zu ermitteln. Zweitens könnte der Drucksensor 300 als schaltbare Komponente ausgeführt sein. Sobald der erste Beschleunigungssensor 2 den Schlagbeginn (über Schwellwert, Kurvencharaceristik, etc.) erkennt, versetzt dieser oder die Recheneinheit 4 den Drucksensor 300 vom ersten Betriebsmodus in den zweiten Betriebsmodus, sodass ab diesem Zeitpunkt Daten von diesem ausgelesen werden können, aus denen die Schlagkraft ermittelt werden kann. Alle vorgenannten Optionen sind somit auch bei der Variante von Figur 9 umsetzbar.

In der Ausführungsform von Figur 9 könnte auch vorgesehen werden, dass zwei Beschleunigungssensoren 2, 3 vorgesehen sind, die in der Regel wie in der erstgenannten Variante betrieben werden. Sobald die Recheneinheit 4 in den zweiten Betriebsmodus versetzt wurde, liest diese nun die aktuellen Messdaten sowohl des zweiten Beschleunigungssensors 3 als auch des Drucksensors 300 aus.

Es versteht sich, dass mittels der beschriebenen Vorrichtung 1 auch weitere, an sich bekannte Funktionen implementiert werden können, wie z.B. das Ermitteln der Schwerkraft durch die im Ruhezustand gemessene Beschleunigung in x-Richtung und Bereinigen der Beschleunigung während eines Schlages oder Aufpralls um die Schwerkraft.

## Patentansprüche

1. Vorrichtung (1) zur Bestimmung einer Schlageigenschaft, insbesondere einer Schlagbeschleunigung, Schlaggeschwindigkeit, Schlagkraft oder Schlagtechnik, umfassend eine Recheneinheit (4), einen ersten Beschleunigungssensor (2) und zumindest einen zweiten Sensor, der bevorzugt ein zweiter Beschleunigungssensor (3) oder ein Drucksensor (300) in einem fluidgefülltem Körper (200) ist,
**dadurch gekennzeichnet, dass**
die Recheneinheit (4) sowohl mit dem ersten Beschleunigungssensor (2) als auch mit dem zweiten Sensor verbunden ist,
wobei die Recheneinheit (4) und/oder der zweite Sensor als schaltbare Komponente ausgeführt und von einem ersten Betriebsmodus in einen zweiten Betriebsmodus versetzbar ist, wobei die schaltbare Komponente im ersten Betriebsmodus einen geringeren Strombedarf aufweist als im zweiten Betriebsmodus, und
wobei der erste Beschleunigungssensor (3) oder der zweite Sensor dazu ausgebildet ist, die schaltbare Komponente vom ersten Betriebsmodus in den zweiten Betriebsmodus zu versetzen, wenn der erste Beschleunigungssensor (2) oder der zweite Sensor Messwerte mit einer vorbestimmten Eigenschaft erkennt, die insbesondere dadurch gegeben sein kann, dass die Beschleunigungsmesswerte des ersten Beschleunigungssensors (2) oder des zweiten Beschleunigungssensors einen Schwellwert (S) überschreiten oder dass die Messwerte einer vorbestimmten Kurvencharakteristik entsprechen.

2. Vorrichtung (1) nach Anspruch 1, wobei der zweite Beschleunigungssensor (3) dazu ausgebildet ist, höhere Beschleunigungsmesswerte als der erste Beschleunigungssensor (2) zu messen.

3. Vorrichtung (1) nach Anspruch 1 oder 2, wobei der erste Beschleunigungssensor (2) einen internen Speicher (20) aufweist und dazu ausgebildet ist, die im internen Speicher (20) gespeicherten Daten auszugeben, wenn die schaltbare Komponente vom ersten Betriebsmodus in den zweiten Betriebsmodus versetzt wird.

4. Vorrichtung (1) nach einem der vorhergehenden Ansprüche, wobei die Recheneinheit (4) die schaltbare Komponente ist und im ersten Betriebsmodus abgeschaltet oder in einem Standby-Modus ist.

5. Vorrichtung (1) nach den Ansprüchen 2, 3 und 4, wobei die Recheneinheit (4) dazu ausgebildet ist, nach dem Versetzen vom ersten Betriebsmodus in den zweiten Betriebsmodus die Daten des internen Speichers (20) des ersten Beschleunigungssensors (2) zu empfangen und beginnend ab dem Zeitpunkt (t0) des genannten Versetzens Beschleunigungsmesswerte des zweiten Beschleunigungssensors (3) zu empfangen, bevorzugt über einen vorbestimmten Zeitraum oder bis ein Beschleunigungsschwellwert unterschritten wird.

6. Vorrichtung (1) nach einem der vorhergehenden Ansprüche, wobei der zweite Beschleunigungssensor (3) als schaltbare Komponente ausgeführt ist und optional gleichzeitig mit der Recheneinheit (4) in den zweiten Betriebsmodus versetzbar ist.

7. Vorrichtung (1) nach Anspruch 6, wobei sich der Schwellwert (S) unterhalb eines maximal messbaren Beschleunigungsmesswertes des ersten Beschleunigungssensors (2) befindet.

8. Vorrichtung (1) nach einem der vorhergehenden Ansprüche, ferner umfassend einen Onboard-Speicher, in dem die Daten des internen Speichers des ersten Beschleunigungssensors und die vom zweiten Beschleunigungssensor empfangenen Daten hinterlegt werden, wobei der Onboard-Speicher über eine externe Schnittstelle auslesbar ist.

9. Vorrichtung nach einem der vorhergehenden Ansprüche, wobei jener Beschleunigungssensor, der dazu ausgebildet ist, höhere Beschleunigungsmesswerte als der andere Beschleunigungssensor zu messen, eine höhere Samplingfrequenz aufweist als der andere Beschleunigungssensor.

10. Vorrichtung (1) nach einem der vorhergehenden Ansprüche, wobei der erste Beschleunigungssensor (2) dazu ausgebildet ist, Beschleunigungsmesswerte von bis zu 16 g in drei orthogonal zueinanderstehenden Raumrichtungen zu messen, und/oder wobei der zweite Beschleunigungssensor (3) dazu ausgebildet ist, Beschleunigungsmesswerte von bis zu 64 g in drei orthogonal zueinander stehenden Raumrichtungen zu messen.

11. Vorrichtung (1) nach einem der vorhergehenden Ansprüche, wobei die genannte Kurvencharakteristik ein sprunghafter Anstieg der gemessenen Beschleunigungswerte ist.

12. Vorrichtung (1) nach einem der vorhergehenden Ansprüche, wobei der erste Beschleunigungssensor (2) oder der zweite Beschleunigungssensor (3) Teil einer inertialen Messeinheit mit einem Drehratensensor zur Bewegungsnachverfolgung ist.

13. Vorrichtung (1) nach einem der vorhergehenden Ansprüche, umfassend ein Kunststoffgehäuse (5), in dem die beiden Beschleunigungssensoren (2, 3) und die Recheneinheit (4) umschlossen sind, wobei die Vorrichtung bevorzugt weiters eine Bandage umfasst, in der das Kunststoffgehäuse (5) aufnehmbar ist, wobei die Bandage bevorzugt eine Tasche aufweist, in der das Kunststoffgehäuse (5) aufnehmbar ist.

14. Vorrichtung (1) nach einem der Ansprüche 1 bis 12, umfassend einen Schlaghandschuh (100) oder einen Körperschutzteil wie einen Kopfschutz.

15. Vorrichtung nach Anspruch 14, wobei der Schlaghandschuh (100) den fluidgefüllten Körper (200) umfasst, in dem der Drucksensor (300) angeordnet ist.
